# EUROPÄISCHE PATENTSCHRIFT

(11) **EP 1 303 294 B1**
(45) Veröffentlichungstag und Bekanntmachung des Hinweises auf die Patenterteilung: **26.07.2006**
(21) Anmeldenummer: 01960527.8
(22) Anmeldetag: 20.07.2001
(51) Int. Cl.: A61K 38/17, A61K 38/16, A61P 9/10

(54) **VERFAHREN ZUR BEHANDLUNG VON INSTABILER ANGINA PECTORIS**
METHOD FOR TREATING UNSTABLE ANGINA PECTORIS
PROCEDE DE TRAITEMENT D'UNE ANGINE DE POITRINE INSTABLE

(30) Priorität: 20.07.2000 DE 10035352
(43) Veröffentlichungstag der Anmeldung: 23.04.2003
(73) Patentinhaber: ZLB Behring AG, 3014 Bern (CH)
(72) Erfinder: LÜSCHER, Thomas, F., CH-8126 Zumikon (CH); NOLL, Georg, CH-8703 Erlenbach (CH); LERCH, Peter, CH-3007 Bern (CH)
(74) Vertreter: Weiss, Wolfgang
(86) Internationale Anmeldenummer: PCT/EP2001/008425
(87) Internationale Veröffentlichungsnummer: WO 2002/007753

(56) Entgegenhaltungen:
- WO-A-01/13939
- WO-A-01/38395
- US-A- 5 128 318
- DIMAYUGA, PAUL ET AL: "Reconstituted HDL Containing Human Apolipoprotein A-1 Reduces VCAM-1 Expression and Neointima Formation Following Periadventitial Cuff-Induced Carotid Injury in apoE Null Mice" BIOCHEMICAL AND BIOPHYSICAL RESEARCH COMMUNICATIONS (1999), 264(2), 465-468 , XP002218783
- SPIEKER, LUKAS E. (1) ET AL: "High-density lipoprotein restores endothelial function in hypercholesterolemic men." CIRCULATION, (OCTOBER 31, 2000) VOL. 102, NO. 18 SUPPLEMENT, PP. II.315-II.316. PRINT. MEETING INFO.: ABSTRACTS FROM SCIENTIFIC SESSIONS 2000 NEW ORLEANS, LOUISIANA, USA NOVEMBER 12-15, 2000 , XP008009388
- LERCH P G ET AL: "Reconstituted high density lipoprotein ( rHDL ) modulates platelet activity in vitro and ex vivo." THROMBOSIS AND HAEMOSTASIS, (1998 AUG) 80 (2) 316-20. , XP008009389
- MOUDRY R ET AL: "Reconstituted high density lipoprotein modulates adherence of polymorphonuclear leukocytes to human endothelial cells." SHOCK, (1997 MAR) 7 (3) 175-81. , XP008009425

## Beschreibung

Die Erfindung betrifft die Verwendung von rekonstituiertem HDL zur Verbesserung der Endothelfunktion bei Hypercholesterinämie und zur Therapie oder Prophylaxe von akuten Koronarerkrankungen wie instabiler Angina pectoris oder Myokardinfarkt.

Das Endothel trennt einerseits das Blut vom extravaskulären Gewebe und reguliert andererseits wichtige Funktionen der Gefässwand. Eine wichtige Voraussetzung für die volle Funktion des endothelialen Monolayers ist dessen funktionelle und strukturelle Integrität. Einige der wichtigen Funktionen des Endothels sind die Steuerung der Adhäsion von Zellen (Monozyten, Plättchen), die Invasion immunkompetenter Zellen und die Proliferation von glatten Muskelzellen. Weiter werden in den Endothelzellen biologisch aktive Substanzen produziert wie Prostaglandine, Stickstoffmonoxid (NO) oder Endothelin-1. Diese Substanzen wirken auf die Struktur, den Metabolismus und die Permeabilität von Gefäßen, modulieren den Gefässtonus und kontrollieren Gerinnung, Fibrinolyse und Entzündungsreaktionen.

Verletzungen des Endothels oder Entzündungen, beispielsweise bedingt durch ungünstige Lipidprofile, führen zu einer Fehlfunktion, einem kritischen Faktor bei der Entwicklung der Atherosklerose. Dieser Vorgang kann über Jahre oder Jahrzehnte ablaufen und führt im Frühstadium zu keinerlei klinischen Symptomen. Strukturelle Veränderungen in der Gefässwand jedoch können mit geeigneten Methoden in diesem Stadium festgestellt werden. Hohe Cholesterinspiegel, insbesondere hohes LDL-Cholesterin bzw. tiefes HDL-Cholesterin begünstigt den raschen Aufbau der Atherosklerose, welche normalerweise mit Ablagerungen in Form von Fatty Streaks beginnt, welche sich später zu Plaques entwickeln können. Solche Plaques können lokal zu vermindertem Blutfluss führen, oder bei erhöhtem mechanischem Stress auch reissen, Thromben bilden und zu akuten koronaren Syndromen führen wie instabile Angina pectoris oder Myokardinfarkt. Die instabile Angina pectoris wird auch als Entzündungsreaktion beschrieben, welche zu einer systematischen Fehlfunktion der Endothelzellen führt.

Die Therapie der akuten koronaren Syndrome versucht die ungenügende Funktion der Endothelzellen wiederherzustellen bzw. zu ersetzen. Das heisst, es wird eine koronare Vasodilatation angestrebt, eine Hemmung der Plättchen, eine Hemmung der Gerinnung oder Revaskularisierung (Bypass, PTCA/Stenting). Dazu werden als pharmazeutische Mittel GP IIb-IIIa Antagonisten, Heparin, Cox-Inhibitoren oder NSAIDs (Aspirin), Betablocker und Nitrate eingesetzt. Langfristig wird versucht, den Plasmacholesterinspiegel zu senken. Mit aggressiver Therapie können akute koronare Syndrome normalerweise innerhalb von 48 Stunden stabilisiert werden.

Wie schon erwähnt, ist ein Risikofaktor, welcher zu Atherosklerose oder akuten koronaren Syndromen führen kann, ein erniedrigter HDL-Cholesterinspiegel. Es wird vermutet, dass HDL mit dem sogenannten reverse Cholesterintransport überflüssiges Cholesterin aus der Peripherie in die Leber führt, dort wird Cholesterin zu Gallensäuren verarbeitet und anschliessend via Galle ausgeschieden. Bei erniedrigten Plasma HDL-Konzentrationen wird Cholesterin zu langsam eliminiert und kann sich in Gefäßwände ablagern.

Die endotheliale Funktion kann in vivo mit verschiedenen Methoden gemessen werden. Die Koronarzirkulation kann mit Katheterisierung und neuen Imaging Techniken quantitativ erfasst werden. Besser und einfacher zugänglich ist die Messung der Zirkulation im Unterarm. Obschon in dortigen Gefäßen normalerweise keine Läsionen beobachtet werden, können mit dieser Methode selbst geringe Änderungen der vaskulären Funktionen beobachtet werden. Mit der Plethysmographie können geringe Änderungen des Blutflusses auf Stimulation mit Acetylcholin bestimmt werden. Mit neuen, nichtinvasiven Ultraschallsystemen kann der Durchmesser von Arterien genau bestimmt werden und so die Gefässfunktion überprüft und beurteilt werden.

Rekonstituiertes HDL kann aus Plasma, Apolipoprotein A-1 und Sojabohnenlecithin gewonnen werden. Verfahren zur Gewinnung von rekonstituiertem HDL (rHDL) sind beispielsweise in Circulatory Shock 40 (1993), 14 - 23, oder in den US Patenten 5,089,602, 5,128,318 und 5,652,339 beschrieben. rHDL bindet an Lipide oder lipidartige Substanzen, beispielsweise Lipopolysaccharide von gram-negativen Bakterien im Blut und kann somit zur Behandlung von Septischem Schock eingesetzt werden.

In einem Abstract von Newton (Workshop: New Aspects of Pharmacological Treatment, Juni 2000) wird offenbart, dass in Tiermodellen für Restenose und Arteriosklerose eine günstige Wirkung bei der Verabreichung von rHDL gefunden wurde. Die Behandlung akuter und chronischer vaskulärer Erkrankungen beim Menschen wird hingegen ausdrücklich als "unerforschtes Gebiet" bezeichnet. Ein möglicher Nutzen bei der Verabreichung von rHDL wird für den reversen Cholesterintransport gesehen, der jedoch für die Bekämpfung einer akuten Erkrankung , wie instabiler Angina pectoris, nicht von Bedeutung ist. Es findet sich somit keinerlei Hinweis, dass rHDL zur kurzfristigen Stabilisierung akuter koronarer Syndrome verwendet werden könnte. Sirtori (Workshop: New Aspects of Pharmacological Treatment, Juni 2000) schlägt die Verwendung von Apolipoprotein-Mutanten und Mimetika zur Behandlung von vaskulären Erkrankungen vor. Es wird berichtet, dass die Verabreichung von rHDL Atherosklerose, Intimaproliferation und Restenose in mehreren Tiermodellen hemmt und die Endothelfunktion in einem Tiermodell, der apoE-KO-Maus, verbessert. An Tiermodellen erhobene Daten können aber nicht ohne weiteres auf den Menschen übertragen werden, aufgrund der z.T. erheblichen Unterschiede im Lipoproteinstoffwechsel bei Nagern und Menschen. Mit dem heutigen Stand der Technik können überdies komplexe Erkrankungen der Koronargefäße, wie Angina pectoris in Tiermodellen nicht nachgeahmt werden. Außerdem findet sich auch keinerlei Hinweis, dass die Verabreichung von rHDL eine kurzfristige Wirkung bei akuten Koronarerkrankungen haben könnte.

In den zur vorliegenden Erfindung führenden Untersuchungen wurde rHDL an Patienten mit erhöhtem Plasmacholesterinspiegel und somit einem erhöhten Risiko für akute koronare Syndrome wie instabile Angina pectoris oder Myokardinfarkt verabreicht. Während der Infusion von rHDL wurde bei den Patienten die Endothelialfunktion in der Unterarmzirkulation verfolgt. Dabei wurde überraschenderweise festgestellt, dass eine sehr schnelle, akute Verbesserung einer vorher gestörten endothelialen Funktion nach Infusion von rHDL erzielt werden kann. Dieser beobachtete akute Effekt von rHDL ist unerwartet und nicht mit den heutigen Vorstellungen über die biologische Wirkung von rHDL beim reversen Cholesterintransport erklärbar oder kompatibel. So kann eine Infusion von rHDL nicht innerhalb der kurzen Versuchsdauer von wenigen Stunden die über Jahrzehnte aufgebauten Auswirkungen der Atherosklerose wie Fatty Streaks oder Plaques rückgängig machen. Vielmehr wird angenommen, dass es sich um einen bisher unbekannten, akuten systemischen Effekt handelt, welcher sehr schnell die gestörte Funktion der Endothelzellen verbessern kann und somit zur Behandlung und/oder Prophylaxe von Koronarerkrankungen, insbesondere instabile Angina pectoris oder Myokardinfarkt, genutzt werden kann.

Die Vorteile einer Therapie von akuten koronaren Syndromen mit rHDL sind eine schnelle Verbesserung der endothelialen Funktion, welche eine günstige Wirkung auf mehrere klinisch relevante Parameter nach sich zieht. Zudem könnte eine nachhaltige Wirkung eintreten, indem ein günstiger Einfluss auf das Lipidprofil erreicht wird. Darüber hinaus handelt es sich bei rHDL um eine körperähnliche Substanz, so dass die Behandlung weitgehend ohne Nebenwirkungen ist. Es wird angenommen, dass die Wirkung von rHDL auf einer verbesserten Bioverfügbarkeit von NO in der Gefässwand beruht, die zu einer Hemmung der Plättchenadhäsion und - aggregation führt und den Gefäßtonus relaxiert.

Nach einer oder mehreren Infusionen von rHDL kann in Patienten mit akuten koronaren Erkrankungen, wie etwa instabiler Angina pectoris, eine geringere Inzidenz der Folgekomplikationen erreicht werden.

Instabile Angina pectoris ist ein klinisches Syndrom myokardialer Ischämie, das durch pektanginöse Thoraxschmerzen in Ruhe mit EKG-Veränderungen charakterisiert wird. Die Abgrenzung zum Myokardinfarkt erfolgt durch biochemische Marker der Myokardschädigung (CK, CK-MB) und EKG.

Ein Gegenstand der vorliegenden Erfindung ist somit die Verwendung von rekonstituiertem HDL zur Herstellung eines Mittels zur Verbesserung der Endothelfunktion, insbesondere eines Mittels zur Verbesserung der akuten Endothelfunktion bei Gefäßerkrankungen, z.B. der akuten Funktion des Koronarendothels, insbesondere in der Humanmedizin.

Ein weiterer Gegenstand der Erfindung ist die Verwendung von rekonstituiertem HDL zur Herstellung eines Mittels zur Prophylaxe oder/und Behandlung akuter Koronarerkrankungen wie etwa instabile Angina pectoris oder Myokardinfarkt. rHDL kann z.B. bei Krankheitsbildern, wie Non Q-Wave-Infarkt und akuten Koronarerkrankungen ohne, mit gering erhöhtem und stark erhöhtem Troponin T Spiegel eingesetzt werden. Besonders bevorzugt wird rHDL zur Verbesserung der endothelabhängigen Vasodilatation bei humanen Patienten eingesetzt.

Die Verabreichung von rHDL erfolgt günstigerweise mittels Infusion, z.B. durch arterielle, intraperitoneale oder vorzugsweise intravenöse Infusion in einer Dosierung von 10 bis 150 mg, vorzugsweise 40 bis 80 mg pro kg Körpergewicht pro Behandlung. Die verabreichte Dosis kann z.B. in einer Menge von 20 bis 100 mg, insbesondere 20 bis 80 mg rHDL entsprechend 0.04 bis 0.6 mg rHDL pro min (Dosis basierend auf Protein) für eine Dauer von 10 min bis mehrere Stunden infundiert werden. Gegebenenfalls kann die rHDL-Infusion bei Bedarf ein oder mehrere Male wiederholt werden.

Das verabreichte rHDL enthält Apolipoprotein A-1 und Phospholipid, z.B. Phosphatidylcholin, vorzugsweise in einem molaren Verhältnis von 1 :50 bis 1:250, besonders bevorzugt in einem Verhältnis von etwa 1:150 und kann zusätzlich weitere Lipide wie etwa Cholesterin enthalten, vorzugsweise bis zu einem molaren Verhältnis von 1:20, z.B. 1:5 bis 1:20 (gegenüber dem Apolipoprotein).

Die rHDL-Verabreichung kann mit der Gabe weiterer Therapeutika wie etwa GP IIb-IIIa Antagonisten, z.B. Antikörpern wie RheoPro, Integrilin oder Tirofiban, Heparin, COX-Inhibitoren oder NSAIDs, wie Aspirin, Betablockern oder Nitraten, kombiniert werden. Besonders bevorzugt ist eine kombinierte Verabreichung mit Heparin oder/und Aspirin.

Schließlich betrifft die Erfindung die Verwendung zur Prophylaxe oder/und Behandlung von akuten Koronarerkrankungen bzw. Koronarsyndromen, wie etwa instabile Angina pectoris oder Myokardinfarkt, wobei man rHDL verabreicht. Die Verabreichung erfolgt einem Subjekt, vorzugsweise einem humanen Patienten derart, dass eine zur Linderung oder Beseitigung akuter Koronarsyndrome ausreichende Menge an rHDL in geeigneter Form, beispielsweise durch Infusion verabreicht wird.

Weiterhin soll die Erfindung durch die folgenden Figuren und Beispiele erläutert werden. Es zeigen:

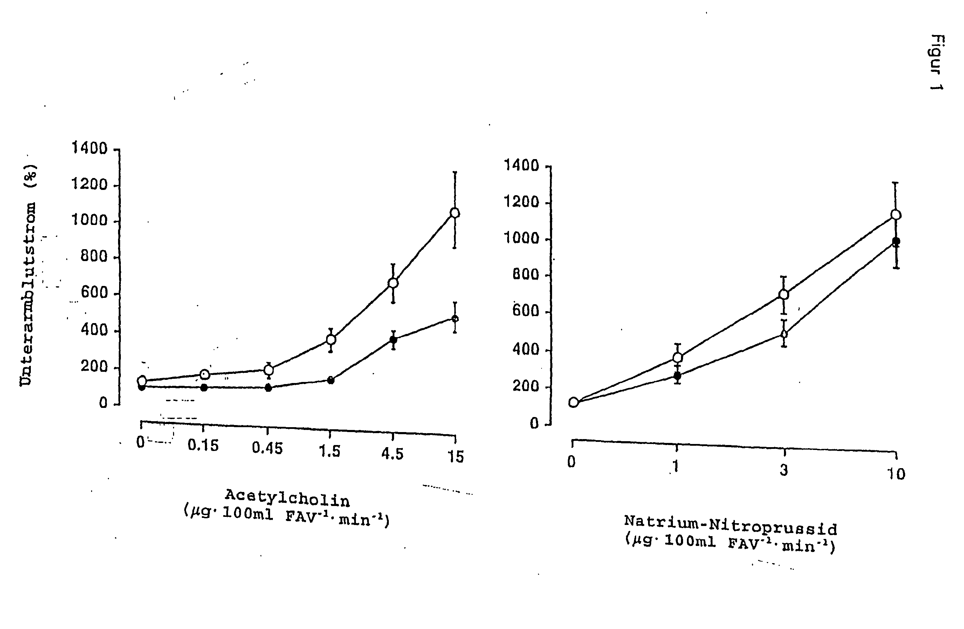

- Figur 1:: Die Reaktionen des Unterarmblutstroms auf intraarterielle Infusion von Acetylcholin oder Natriumnitroprussid in normalen oder hypercholesterinämischen Personen. Die endothelabhängige Vasodilatation auf Acetylcholin ist in hypercholesterinämischen Personen (geschlossene Kreise) im Vergleich zu gesunden Probanden (offene Kreise) signifikant gehemmt (p < 0,0001). Die endothelunabhängige Vasodilatation auf Natriumnitroprussid ist in beiden Gruppen vergleichbar.

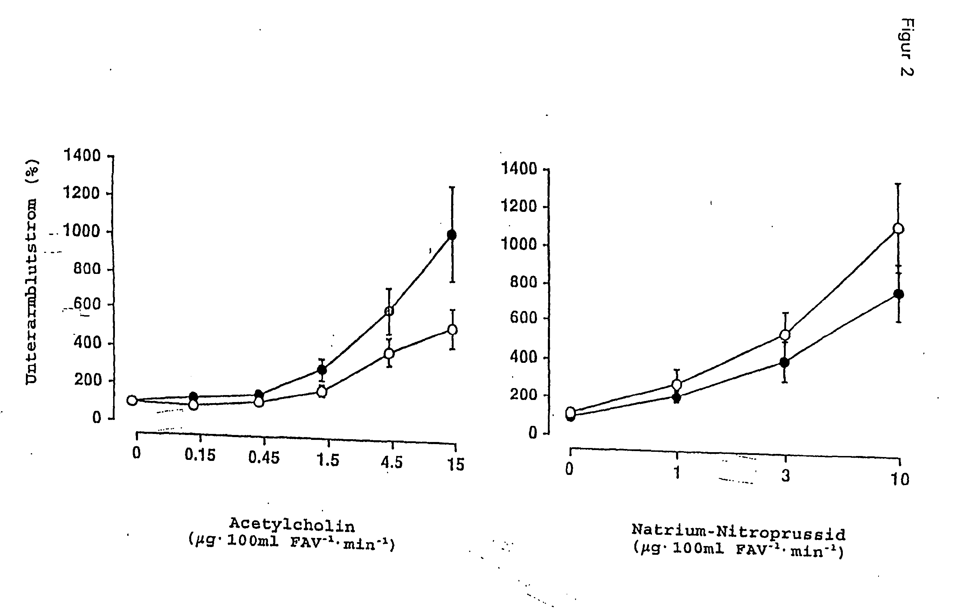

- Figur 2:: Die endothelabhängige- und -unabhängige Vasodilatation auf Acetylcholin oder Natriumnitroprussid in hypercholesterinämischen Personen vor (offene Kreise) oder nach (geschlossene Kreise) intravenöser Infusion von rekonstituiertem HDL. Es gibt eine signifikante Verbesserung (p =0,017) bei der endothelabhängigen Vasodilatation auf Acetylcholin, nicht aber auf Natriumnitroprussid, einem endothel-unabhängigen Vasodilator.

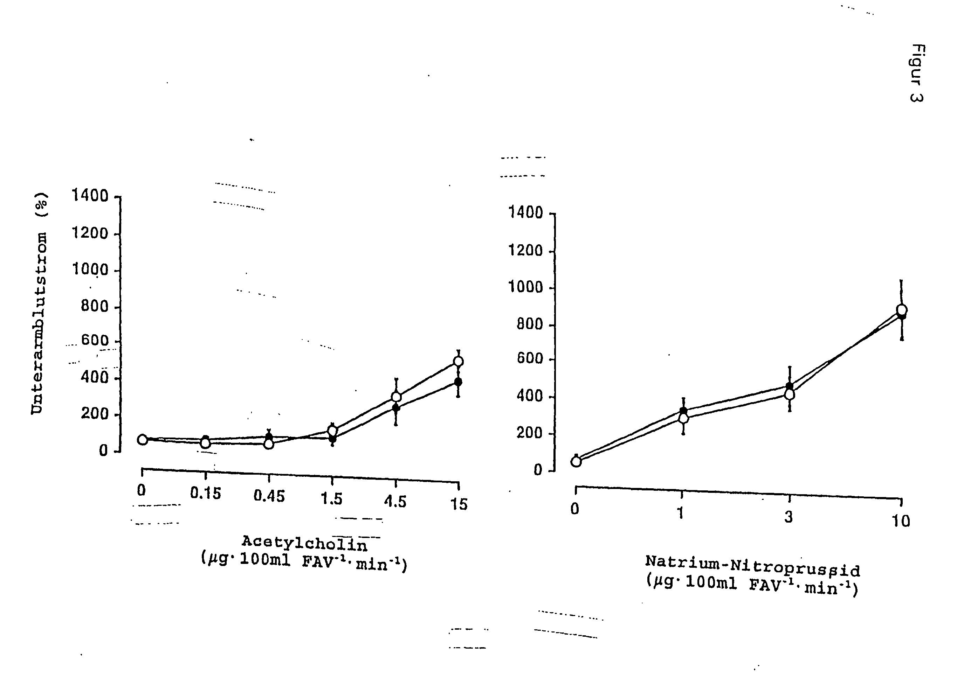

- Figur 3:: Die endothelabhängige und -unabhängige Vasodilatation nach Verabreichung von Acetylcholin oder Natriumnitroprussid in hypercholesterinämischen Patienten vor (offene Kreise) und nach (geschlossene Kreise) intravenöser Infusion von rekonstituiertem HDL während einer Hemmung der NO-Synthase mit L-NMMA. Es gibt keine signifikante Änderung in den Reaktionen auf intraarterielle Infusionen mit Acetylcholin oder Natriumnitroprussid.

### Beispiel 1

### 1. Methoden

### 1.1 Patienten

18 gesunde, hypercholesterinämische Männer mit einer Plasma-LDL-Cholesterinkonzentration > 155 mg/dl (> 4,0 mmol/l) und 8 gesunde, normocholesterinämische Männer mit einem LDL-Cholesteringehalt < 135 mg/dl (< 3,5 mmol/l) nahmen an der Untersuchung teil. Ausschlusskriterien waren arterieller Bluthochdruck (≥ 140/90 mmHg), Diabetes mellitus und Rauchen.

Die Patienten nahmen während der letzten 12 Stunden vor der Untersuchung weder Nahrung, Alkohol, noch Koffein zu sich. Eine Statintherapie wurde bei zwei Patienten vier Wochen vor dem Experiment abgesetzt, alle anderen Personen nahmen keine lipidsenkenden Mittel oder andere Mittel zu sich.

### 1.2 Protokoll

Der Unterarmblutstrom (Vorderarmblutfluss) wurde gleichzeitig in beiden Armen durch venöse Okklusionsplethysmographie (EC-4, Hokanson) bestimmt (Benjamin et al, Hypertension 25 (1995), 918-923). Die Personen lagen ausgestreckt mit leicht angehobenen Armen über der Höhe des Herzens, um die venöse Drainage zu verbessern. Eine Handzirkulation wurde durch Handgelenksbandagen mit suprasystolischem Druck (220 mmHg) eine Minute vor den Messungen verhindert (Kerslake, J. Physiol. 108 (1949), 451-457). Zur Bestimmung der endothelabhängigen und - unabhängigen Vasodilatation wurden Acetylcholin (Miochol E, Ciba Vision, Schweiz) und Natriumnitroprussid (Nipruss, Schwarz Pharma, Deutschland) in die Brachialarterie in zunehmenden Mengen von 0,15, 0,45, 1,5, 4,5 und 15 *µ*g x 100 ml Unterarmvolumen (FAV)⁻¹ x min⁻¹ (jeweils in 0,9 % NaCl für 5 Minuten) und 1,3 und 10 *µ*g x 100 ml FAV⁻¹ x min⁻¹ (in 5 % Glucose für 3 Minuten) mit konstanter Geschwindigkeit durch eine Infusionspumpe über einen unter Lokalanästhesie eingeführten Katheter infundiert. Zwischen den intraarteriellen Infusionen wurde jeweils eine Pause von 30 Minuten gelassen. Die Signale wurden mit einem Computer (PowerBook G3; Apple Macintosh) unter Verwendung eines Analog/Digital Board (National Instruments) und einer modifizierten kommerziellen Datenerfassungsoftware (LabView, National Instruments) aufgezeichnet.

In hypercholesterinämische Freiwillige wurden rekonstituierte PDL-Partikel (rHDL, ZLB Bioplasma AG, Bern, Schweiz; n = 7) enthaltend Apolipoprotein A-1 und Phosphatidylcholin in einem Verhältnis von 1:154 oder Albumin (5 %, ebenfalls vom ZLB; n = 5) intravenös in einer äquivalenten Proteindosierung von 80 mg/kg über vier Stunden infundiert (Lerch et al, Vox Sang 71 (1996), 155 - 164; Pajkrt et al, J. Exp. Med. 184 (1996), 1601 - 1608; Pajkrt et al, Thromb. Haemostat. 77 (1997), 303 - 307). Dann wurden die Infusionen von Acetylcholin und Natriumnitroprussid wiederholt.

Bei 5 weiteren hypercholesterinämischen Männern wurden die Wirkungen einer intravenösen rHDL-Infusion auf die Acetylcholin- und Natriumnitroprussid-induzierte Vasodilatation während einer intraarteriellen Coinfusion von N^{G}-Monomethyl-L-Arginin (L-NMMA, 4 *µ*mol/min; Clinalfa), einem Inhibitor der NO-Synthase, bestimmt. Der intraarterielle Blutdruck und der Herzschlag wurden kontinuierlich aufgezeichnet.

### 1.3 Biochemische Analysen

Blutproben wurden vor oder nach Infusion von rHDL bzw. Albumin entnommen. Gesamtcholesterin, HDL-Cholesterin, Triglyceride, Lebertransaminasen und Insulin wurden nach Standardlabormethoden bestimmt. Der LDL-Cholesteringehalt wurde unter Verwendung der Friedewald-Formel berechnet (Friedewald et al, Clin. Chem. 18 (1972), 499 - 502).

### 1.4 Statistische Analyse

Die Ergebnisse werden als Mittelwerte ± Standardabweichung (SD) angegeben. Die durchschnittlichen Werte für den Unterarmblutstrom wurden aus mindestens drei aufeinander folgenden Aufzeichnungen während der letzten Minute der Arzneimittelinfusion erhalten. Sie sind als prozentuales Verhältnis des infundierten zum nicht infundierten Arm angegeben, um Veränderungen im Blutstrom, verursacht durch systemische Faktoren, zu berücksichtigen. Plethysmographische Messungen wurden unter Verwendung von Zweiweg-ANOVA für wiederholte Messungen verglichen (Wallenstein und Zucker, Circ. Res. 47 (1980), 1 - 9). Ein Vergleich der klinischen Daten erfolgte durch den gepaarten oder ungepaarten Student-T-Test (StatView 4.5, Abacus Konzept). Eine statistische Signifikanz wurde bei P < 0,05 angenommen.

### 2. Ergebnisse

Die klinischen Merkmale der Teilnehmer an der Untersuchung sind in Tabelle 1 gezeigt. Die hypercholesterinämischen und normocholesterinämischen Untersuchungsgruppen unterschieden sich nur in den Lipidkonzentrationen und dem Körpermassenindex. Es gab keinen Unterschied im basalen Unterarmblutstrom in den beiden Gruppen. Die vasodilatatorische Reaktion auf Acetylcholin, nicht aber auf Nitroprussid, war bei den hypercholesterinämischen Männern signifikant verringert (Fig. 1).

Die Wirkungen von rHDL- bzw. Albumininfusionen auf Laborparameter und hämodynamische Parameter der hypercholesterinämischen Männer sind in Tabelle 2 gezeigt. Es gab keine statistisch signifikanten Unterschiede zwischen den beiden hypercholesterinämischen Gruppen, denen rHDL bzw. Albumin verabreicht wurde. Die intravenöse cholesterinfreie rHDL-Infusion führte zu einer signifikanten Erhöhung der Plasma-HDL-Cholesterinkonzentration, nicht aber der LDL-Cholesterin-, Triglycerid- oder Insulinplasmakonzentration. Die endothelabhängige Vasodilatation nach Verabreichung von Acetylcholin wurde durch rHDL-Infusion signifikant erhöht, während die endothelunabhängige Vasodilatation auf Natriumnitroprussid unverändert blieb (Fig. 2 und Tab. 3).

Die rHDL-induzierte Verbesserung in der Vasodilatation auf Acetylcholin wurde durch gleichzeitige intraarterielle Infusion von L-NMMA verhindert (Fig. 3 und Tab. 3). Die endothelunabhängige Vasodilatation auf Natrium nitro prussid wurde durch L-NMMA nicht beeinflusst. L-NMMA führte zu einer signifikanten Verringerung des basalen Unterarmblutstroms bei hypercholesterinämischen Personen (- 23 ± 3 %). Die vasodilatatorischen Reaktionen auf Acetylcholin und Natriumnitroprüssid unterschieden sich nicht signifikant in der L-NMMA Infusionsgruppe verglichen mit der hypercholesterinämischen Gruppe ohne gleichzeitige L-NMMA Infusion.

Die Albumininfusion beeinflusste weder die vasodilatatorische Reaktion auf Acetylcholin noch auf Natriumnitroprussid (Tab. 3). Auch sonstige Laborparameter wurden nicht beeinflusst (Tab. 2). Es wurden keine Nebenwirkungen gefunden. Es gab auch keine signifikanten Änderungen im arteriellen Blutdruck oder im Herzschlag während der Untersuchungsdauer. Der Blutstrom im nicht infundierten Kontrollarm wurde durch die intraarteriellen Infusionen nicht verändert. Sowohl in der rHDL als auch in der Albumingruppe zeigte der basale Unterarmblutstrom einen deutlichen, aber nicht statistisch signifikanten Anstieg über die 6-stündige Dauer der Experimente (Tab. 3).

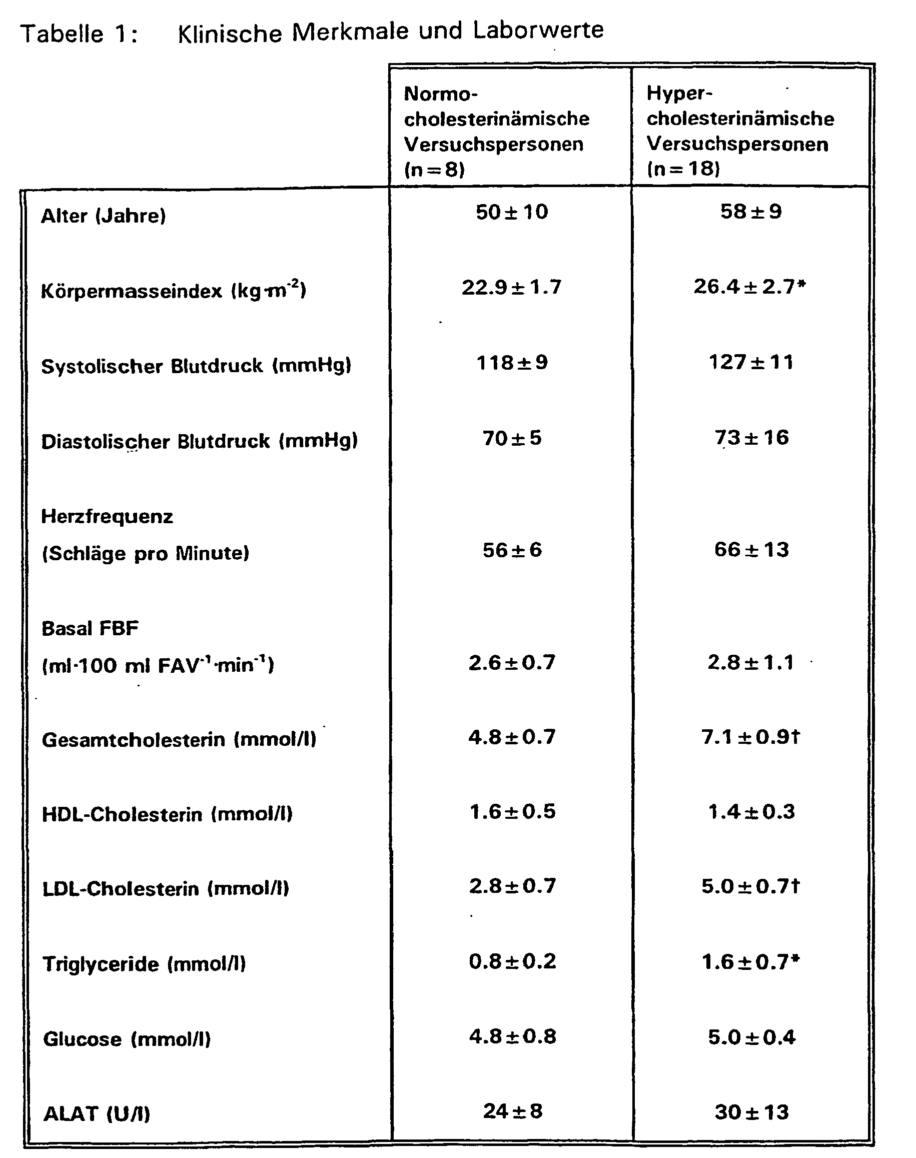

Werte sind Mittelwerte ± SD
FBF bedeutet Unterarmblutstrom, FAV Unterarmblutvolumen und
ALAT Alanin-Aminotransferase.
*P<0.01, †P<0.0001

**Tabelle 2: Wirkung von Infusionen auf die Merkmale von hypercholesterinämischen Versuchspersonen**

| | rHDL (n=12) | | Albumin (n=5) | |
|---|---|---|---|---|
| | prä | post | prä | post |
| Alter (Jahre) | 55 ± 3 | | 58 ± 4 | |
| Körpermasseindex (kg·m⁻²) | 27±1.2 | | 26.0 ± 1.1 | |
| Systolischer Blutdruck (mmHg) | 123 ± 2 | 123 ± 1 | 121 ± 11 | 123 ± 11 |
| Diastolischer Blutdruck (mmHg) | 61 ± 3 | 61 ± 3 | 62 ± 4 | 64 ± 6 |
| Herzschlag (min⁻¹) | 64 ± 3 | 66 ± 3 | 60 ± 5 | 57 ± 5 |
| Gesamtcholesterin (mmol/l) | 6.7 ± 0.3 | 7.4 ± 0.3 | 6.5 ± 0.4 | 6.4 ± 0.4 |
| HDL-Cholesterin (mmol/l) | 1.2 ± 0.0 | 2.1 ± 0.1* | 1.4 ± 0.1 | 1.4 ± 0.1 |
| LDL-Cholesterin (mmol/l) | 5.2 ± 0.3 | 4.9 ± 0.2 | 4.5 ± 0.3 | 4.5 ± 0.4 |
| Triglyceride (mmol/l) | 1.5 ± 0.2 | 2.1 ± 0.3 | 1.2 ± 0.2 | 1.2 ± 0.2 |
| Insulin (pmol/l) | 66 ± 14 | 63 ± 11 | 76 ± 15 | 62 ± 10 |
| Glucose (mmol/l) | 5.0 ± 0.2 | | 5.1 ± 0.2 | |
| Alanin-Aminotransferase (U/I) | 18 ± 1.3 | 18 ± 1.3 | | |

Werte sind Mittelwerte ± SD.
FBF bedeutet Unterarmblutstrom, FAV Unterarmblutvolumen
*P < 0.0001 gegenüber entsprechendem Prä-Infusionswert

**Tabelle 3: Wirkung verschiedener Mittel auf den Unterarmblutstrom bei hypercholesterinämischen Männern**

| | | rHDL (80mg/kg, n=7) | | Albumin (80mg/kg, n=5) | | L-NMMA + rHDL (80mg/kg, n = 5) | |
|---|---|---|---|---|---|---|---|
| | | prä | post | prä | post | prä | post |
| Basiswert | | 3.0±0.5 | 3.3±0.5 | 2.7±0.5 | 3.3±0.8 | 2.0±0.3 | 2.3±0.3 |
| Acetylcholin (*µ*g/min/100ml FAV) | 0.15 | 3.2 ±0.6 | 4.5±0.6* | 3.4±0.7 | 3.5±0.5 | 1.9±0.3 | 2.1±02 |
| | 0.45 | 3.5±0.5 | 4.6±0.6* | 3.4±0.6 | 3.8±0.6 | 2.0±0.2 | 2.0±0.3 |
| | 1.5 | 6.2±1.9 | 9.3±2.0* | 5.6±1.7 | 7.0±2.0 | 3.9±1.0 | 3.4±1.2 |
| | 4.5 | 11.6±2.7 | 17.8±2.8* | 12.9±4.2 | 14.4±5.3 | 8.4±2.2 | 7.5±2.3 |
| | 15 | 14.8±3.0 | 28.3±4.3* | 17.6±6.4 | 20.6±9.1 | 12.2±1.3 | 11.1±1. |
| Basislinie | | 3.2±0.4 | 3.7±0.5 | 2.7±0.3 | 2.5±0.2 | 1.7±0.2 | 2.2±0.3 |
| Nitroprussid (*µ*g/min/100ml FAV) | 1 | 5.9±1.0 | 7.5±0.9 | 9.2±1.8 | 8.0±1.4 | 8.5±2.4 | 124±3 |
| | 3 | 10.7±1.7 | 13.2±2.0 | 14.4±2.8 | 15.3±2.6 | 12.0±3.3 | 13.9±3. |
| | 10 | 22.2±2.2 | 25.6±2.2 | 23.9±3.3 | 22.1±4.1 | 20.5±2.2 | 19.7±2. |

| | | | | | | | |
|---|---|---|---|---|---|---|---|
| Werte sind Mittelwerte ± SD des Unterarmstroms | | | | | | | |
| *P<0.05 für prä gegenüber post | | | | | | | |

### Beispiel 2

### 1. Methoden

### 1.1 Patienten

Patienten mit instabiler Angina pectoris (Braunwald-Klassifikation IIIb) nach erfolgter Akutbehandlung, d.h. 3-5 Tage nach Hospitalisation, nehmen an der Untersuchung teil. Das Alter der Patienten liegt zwischen 20 und 75 Jahren.

### 1.2 Protokoll

Der Unterarmblutstrom wird gleichzeitig an beiden Armen durch venöse Okklusionsplethysmographie bestimmt. Dabei wird mit einer auf 40 mm Hg aufgepumpten Blutdruckmanschette der venöse Blutabfluss am Oberarm verhindert, so dass nur Blut in den Arm hinein - aber nicht hinausströmen kann. Die dadurch bewirkte Zunahme des Unterarmumfanges wird mit einem dehnungssensitiven Gummiband gemessen. Die Handzirkulation wird mit einer Handgelenksmanschette vorübergehend unterbrochen. Die Signalaufzeichnung erfolgt wie in Beispiel 1 beschrieben.

Zur Messung der Endothel-abhängigen und -unabhängigen Vasodilatation wird Acetylcholin bzw. Natriumnitroprussid, wie in Beispiel 1 beschrieben, infundiert.

Für die Positronenemissionstomographie (PET) wird ein GE Advance PET-Tomograph (GE Medical Systems, Milwaukee, Wisconsin) mit einem axialen Gesichtsfeld von 35 x 4,25 mm verwendet. Den Freiwilligen werden 700-900 MBq ¹³N-Ammoniak oder ¹⁵O-Wasser in eine periphere Vene durch Bolusinjektion verabreicht, während serielle transaxiale tomografische Abbildungen des Herzens aufgezeichnet werden (Rahmen: 12 x 10 s, 4 x 30 s, 1 x 60 s und 2 x 30 s). Nach 20minütiger Aufzeichnung wird ein 20minütiger Transmissionsscan zur Photonenattenuierungskorrektur unter Verwendung externer ⁶⁸Ge-Quellen durchgeführt. Der Myokard-Blutstrom (MBF) wird bei Ruhe und unter Standardstressbedingungen, d.h. Gabe von Adenosin (0, 14mg/kg/min über 7 Minuten) gemessen.

Das Herz wird in 16 Segmente gemäß den Empfehlungen der American Society of Echocardiography unterteilt. Außerdem wird der linke ventrikuläre Blutstrom untersucht.

Der MBF wird nach einer bei Muzik et al. (J. Nucl. Med. 34 (1993), 83-91) beschriebenen Methode bestimmt und nach Hutchins (J. Am. Coll. Cardiol. 15 (1990), 1032-1042) und Hutchins et al. (J. Nucl. Med. 33 (1992), 1243-1250) einer Korrektur unterzogen. Die Koronarflussreserve (CFR) wird als Verhältnis zwischen hyperämischen und Ruhe-MBF-Werten berechnet. Die Reproduzierbarkeit dieses Verfahrens wird von Kaufmann et al. (J. Nucl. Med. 40 (1999), 1848-1856) und Wyss et al. (Eur. Heart J. 21 (2000), 568) bestätigt.

Nach einer ersten Messung der Endothelfunktion mittels Plethysmographie und Positronenemissionstomographie wird rHDL in einer Dosierung von 80 mg/kg über 4 Stunden intravenös infundiert und die Endothelfunktion ein zweites Mal gemessen. Blutdruck und Herzfrequenz werden kontinuierlich aufgezeichnet.

## Patentansprüche

1. Verwendung von rekonstituiertem HDL zur Herstellung eines Mittels zur Behandlung von Gefäßkrankheiten für die akute Verbesserung der Endothelfunktion.

2. Verwendung von rekonstituiertem HDL zur Herstellung eines Mittels zur Prophylaxe oder/und Behandlung akuter Koronarerkrankungen.

3. Verwendung nach Anspruch 1 oder 2 zur Prophylaxe oder/und Behandlung von instabiler Angina pectoris oder Myokardinfarkt.

4. Verwendung nach einem der Ansprüche 1-3,
**dadurch gekennzeichnet,**
**dass** die Verabreichung von rHDL durch Infusion erfolgt.

5. Verwendung nach Anspruch 4,
**dadurch gekennzeichnet,**
**dass** eine intravenöse Infusion erfolgt.

6. Verwendung nach einem der Ansprüche 1 - 5,
**dadurch gekennzeichnet,**
**dass** 10 - 150 mg rHDL (Gewicht bezogen auf das Apolipoprotein) pro kg Körpergewicht pro Behandlung verabreicht werden.

7. Verwendung nach Anspruch 6,
**dadurch gekennzeichnet,**
**dass** die verabreichte Dosis in einer Menge von 0.04 bis 0.6 mg rHDL (Gewicht bezogen auf das Apolipoprotein) pro min für eine Dauer von 10 min bis mehrere Stunden infundiert wird.

8. Verwendung nach einem der Ansprüche 1 - 7,
**dadurch gekennzeichnet,**
**dass** das verabreichte rHDL ein molares Verhältnis von Apolipoprotein A-I zu Phospholipiden im Bereich von 1:50 bis 1:250 aufweist, und gegebenenfalls zusätzliche Lipide wie Cholesterin in einem molaren Verhältnis von bis zu 1:20 zum Apolipoprotein enthält.

9. Verwendung nach einem der Ansprüche 1 - 8,
**dadurch gekennzeichnet,**
**dass** die Verabreichung von rHDL mit der Gabe anderer Therapeutika kombiniert wird.

## Claims

1. Use of reconstituted HDL for producing a composition for treating vascular disorders for acute improvement of endothelial function.

2. Use of reconstituted HDL for producing a composition for the prophylaxis or/and treatment of acute coronary disorders.

3. Use according to Claim 1 or 2 for the prophylaxis or/and treatment of unstable angina pectoris or myocardial infarction.

4. Use according to any of Claims 1-3, **characterized in that** rHDL is administered by infusion.

5. Use according to Claim 4, **characterized in that** intravenous infusion takes place.

6. Use according to any of Claims 1-5, **characterized in that** 10-150 mg of rHDL (weight based on the apolipoprotein) are administered per kg of bodyweight per treatment.

7. Use according to Claim 6, **characterized in that** the administered dose is infused in an amount of from 0.04 to 0.6 mg of rHDL (weight based on the apolipoprotein) per min for a time of from 10 min to several hours.

8. Use according to any of Claims 1-7, **characterized in that** the administered rHDL has a molar ratio of apolipoprotein A-I to phospholipids in the range from 1:50 to 1:250, and optionally comprises additional lipids such as cholesterol in a molar ratio of up to 1:20 to the apolipoprotein.

9. Use according to any of Claims 1-8, **characterized in that** administration of rHDL is combined with administration of other therapeutic agents.

## Revendications

1. Utilisation de HDL reconstituée pour la production d'une composition destinée au traitement de troubles vasculaires pour une amélioration de la fonction endothéliale.

2. Utilisation de HDL reconstituée pour la production d'une composition destinée à la prophylaxie et/ou au traitement de troubles coronariens aigus.

3. Utilisation selon la revendication 1 ou 2, pour la prophylaxie et/ou le traitement d'une angine de poitrine instable ou d'un infarctus du myocarde.

4. Utilisation selon l'une quelconque des revendications 1 à 3, **caractérisée en ce que** la rHDL est administrée par perfusion.

5. Utilisation selon la revendication 4, **caractérisée en ce qu'**une perfusion intraveineuse a lieu.

6. Utilisation selon l'une quelconque des revendications 1 à 5, **caractérisée en ce que** 10 à 150 mg de rHDL (poids basé sur l'apolipoprotéine) sont administrés par kg de poids corporel par traitement.

7. Utilisation selon la revendication 6, **caractérisée en ce que** la dose administrée est perfusée en une quantité de 0,04 à 0,6 mg de rHDL (poids basé sur l'apolipoprotéine) par min pendant un temps de 10 min à plusieurs heures.

8. Utilisation selon l'une quelconque des revendications 1 à 7, **caractérisée en ce que** la rHDL administrée présente un rapport molaire de l'apolipoprotéine A-I sur les phospholipides dans la plage de 1/50 à 1/250, et comprend éventuellement d'autres lipides tels que du cholestérol dans un rapport molaire de jusqu'à 1/20 sur l'apolipoprotéine.

9. Utilisation selon l'une quelconque des revendications 1 à 8, **caractérisée en ce que** l'administration de rHDL est combinée à l'administration d'autres agents thérapeutiques.
